Europäisches Patentamt

European Patent Office

Office européen des brevets

(19)

(11) Publication number: **0 122 293**
**B1**

(12) **EUROPEAN PATENT SPECIFICATION**

(45) Date of publication of patent specification: **16.08.89**

(51) Int. Cl.⁴: **A 61 N 1/42**

(21) Application number: **83903224.0**

(22) Date of filing: **17.10.83**

(86) International application number:
**PCT/JP83/00361**

(87) International publication number:
**WO 84/01517 26.04.84 Gazette 84/11**

(54) **MAGNETIC BLANKET.**

(30) Priority: **18.10.82 JP 15718/82**

(43) Date of publication of application:
**24.10.84 Bulletin 84/43**

(45) Publication of the grant of the patent:
**16.08.89 Bulletin 89/33**

(84) Designated Contracting States:
**AT CH DE FR GB LI**

(56) References cited:
**JP-U-54 022 917**
**JP-U-54 025 109**

(73) Proprietor: **Nihon Kenkozoshin Kenkyukai Co., Ltd.**
**1-29 Minato 2-chome Chuo-ku**
**Fukuoka-shi Fukuoka 810 (JP)**

(72) Inventor: **MASUDA, Isamu**
**13-217, Ozasa 3-chome Chuo-ku**
**Fukuoka-shi Fukuoka 810 (JP)**

(74) Representative: **Otto, Dieter Dr. et al**
**Isartorplatz 6 Postfach 260247**
**8000 München 2 (DE)**

## Description

### Background Art

It has been proposed in the past to provide magnets on or in a. sleeping mat. Providing magnets in or on a sleeping mat, however, leads to discomfort when the full weight of the individual presses down upon the magnets for any extended period of time. To prevent this discomfort, such magnets must be embedded so deeply in the mat that the beneficial effects of the magnets are negated.

It is an object of the present invention to provide a bed coverlet on which magnets are mounted, the magnets provided on the coverlet can be close to the human body, thereby enabling full utilization of the magnetic force of the magnets for a long time.

### Disclosure of Invention

This invention relates to a bed coverlet having plurality of magnets mounted thereon, the magnets distributed over an area of the coverlet which corresponds to the figure or shape of a person lying on a mat.

### Brief Description of Drawings

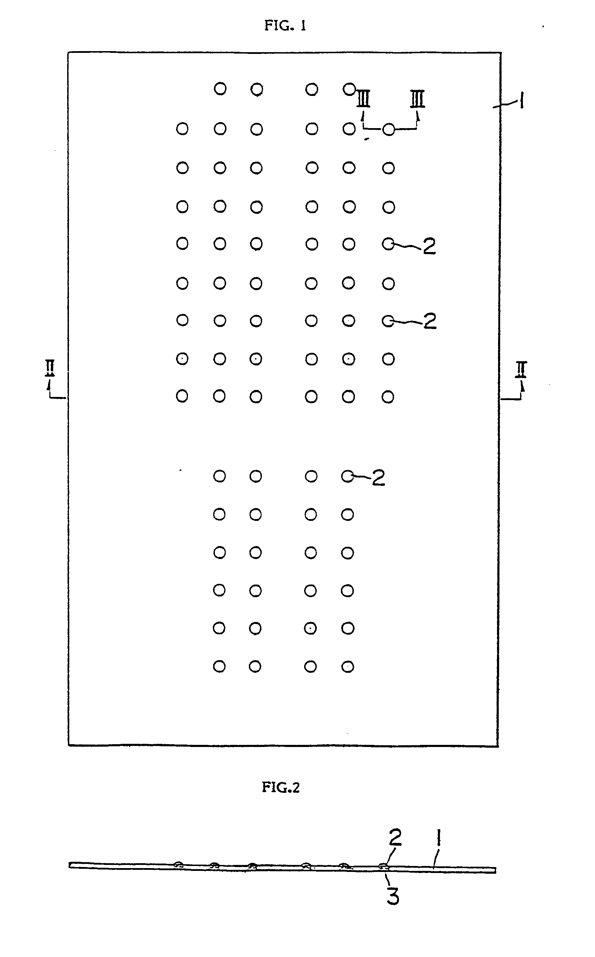

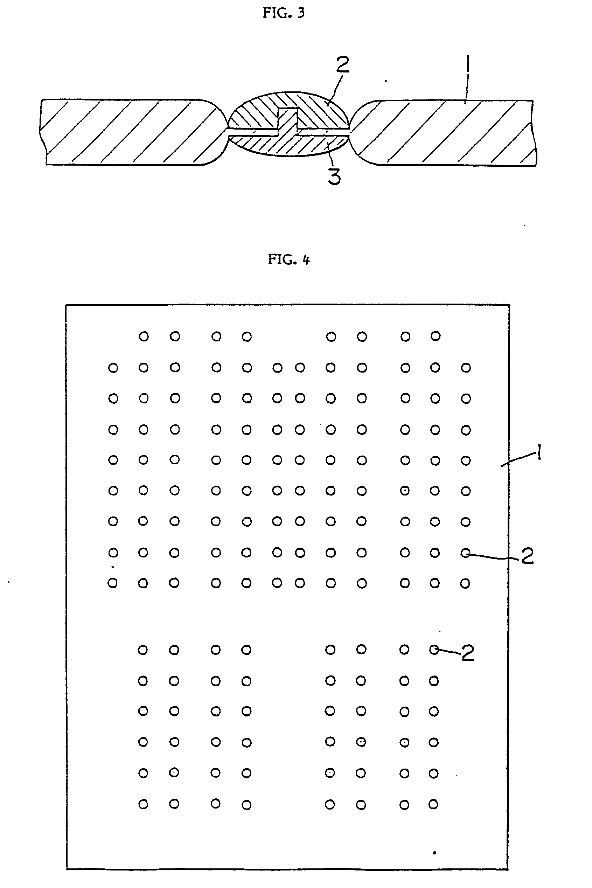

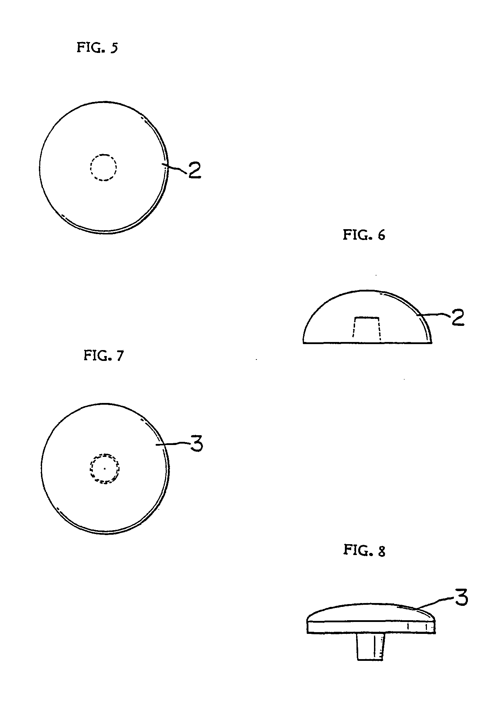

Fig. 1 is a plan view of the bed coverlet of one embodiment of the present invention. Fig. 2 is a front cross sectional view of the bed coverlet taken on the line II—II of Fig. 1. Fig. 3 is an enlarged cross-sectional view of the bed coverlet taken on the line III—III of Fig. 1. Fig. 4 is a plan view of the bed coverlet of the second embodiment of the present invention. Fig. 5 and Fig. 6 are plan view and front view of the magnet used in the bed coverlet of the present invention. Fig. 7 and Fig. 8 are plan view and front view of the magnet mounting bushing used in the bed coverlet of the present invention.

### Best Mode for Carrying Out the Invention

The present invention is hereinafter disclosed in detail in conjunction with the attached drawings.

In Fig. 1 and Fig. 2, a bed coverlet of the present invention for a single bed is disclosed. Magnets (2) are distributed on a coverlet (1) in such a manner that the magnets (2) cover the area which corresponds to the figure or shape of a person sleeping on a sleeping mat. As shown in Fig. 3, the magnet (2) has a spherical front surface and a flat bottom surface. The bottom surface is provided with an opening which cooperates with a magnet mounting bushinbg (3) for mounting the magnets (2) to the coverlet (1).

Extremely soft foamed material is considered to be a suitable material for the coverlet (1), since the material shows an excellent flexibility and is light in weight.

Fig. 4 shows a bed coverlet of the present invention for a double bed.

In Fig. 5 and Fig. 6, the magnet element used for the bed coverlet of the present invention is shown, wherein the spherical front surface thereof is determined as a north pole while the opposite bottom surface is determined to be a south pole. The magnet element is provided with a recess in the flat surface thereof. In Fig. 7 and Fig. 8, the magnet mounting bushing (3) is shown wherein the bushing (3) is provided with a protrusion which snugly engages the recess.

In the above two embodiments, although the magnets (2) are arranged on the coverlet (1) corresponding to the figure of the human body, magnets (2) may be arranged corresponding to the sweet points of oriental medic.

According to the present invention, since the magnets are mounted on the bed coverlet rather than the mat, the magnets provided on the coverlet can be close to the human body, thereby enabling full utilization of the magnetic force of the magnets. Furthermore, since the magnets are specifically designed to cover the human body and not to be laid upon, comfort during sleep is assured.

### Claims

1. A bed coverlet provided with magnets comprising a coverlet and a plurality of magnets mounted on said coverlet, said magnets being distributed over an area of said coverlet which corresponds to the figure or shape of a person lying on a mat.

2. A bed coverlet provided with magnets according to claim 1, wherein said coverlet is made of an extremely soft material.

### Patentansprüche

1. Bettdecke mit Magneten, bestehend aus einer Bettdecke und einer Vielzahl von Magneten, die auf der Bettdecke befestigt sind, wobei die Magnete über eine Fläche der Bettdecke verteilt sind, welche der Figur oder Form einer Person entspricht, die auf einer Matte liegt.

2. Bettdecke mit Magneten nach Anspruch 1, wobei die Bettdecke aus einem extrem weichen Material besteht.

### Revendications

1. Dessus de lit muni d'aimants comprenant un dessus de lit et une série d'aimants montés sur ledit dessus de lit, lesdits aimants étant répartis sur une zone dudit dessus de lit qui correspond à la silhouette ou à la forme d'une personne reposant sur un matelas.

2. Dessus de lit muni d'aimants selon la revendication 1, dans lequel ledit dessus de lit est en une matière extrêmement molle.

FIG. 1

FIG.2

FIG. 3

FIG. 4

FIG. 5

~2

FIG. 6

~2

FIG. 7

~3

FIG. 8

~3